# EUROPEAN PATENT APPLICATION

(11) **EP 4 242 314 A1**
(43) Date of publication of application: **13.09.2023**
(21) Application number: 21889678.5
(22) Date of filing: 09.11.2021
(51) Int. Cl.: C12N 15/74, C07K 14/255, C07K 14/195, A61K 35/74, A61P 35/00

(54) **SALMONELLA STRAIN FOR PREVENTION AND TREATMENT OF CANCER AND USE THEREOF**

(30) Priority: 09.11.2020 KR 20200148824
(71) Applicant: Industry Foundation of Chonnam National University, Gwangju 61186 (KR)
(72) Inventor: MIN, Jung-Joon, Gwangju 61461 (KR); HONG, Yeongjin, Gwangju 61682 (KR); YOU, Sung-Hwan, Gwangju 61419 (KR); HUY, Nguyen Dinh, Hwasun-gun, Jeollanam-do 58116 (KR)
(74) Representative: Thomann, William John
(86) International application number: PCT/KR2021/016267
(87) International publication number: WO 2022/098213

(57) **Abstract**

The present disclosure relates to a DNA construct, and a strain into which a recombinant vector comprising the DNA construct has been introduced. The DNA construct according to the present disclosure allows the expression of anticancer genes, operably linked downstream of first and second promoters, in a host strain or cell, to prevent and treat cancer. In addition, the DNA construct of the present invention can control the treatment of doxycycline so that the anticancer protein can be expressed at an appropriate dose for cancer treatment.

## Description

### Technical Field

The present invention relates to a DNA construct for preventing and treating cancer and a strain into which a recombinant vector comprising the DNA construct has been introduced.

### Background Art

To date, most cancers have been treated by each individual method corresponding to surgery, radiotherapy, chemotherapy, or a combination thereof. Surgical operation that removes most of the cancerous tissue may be very effective at removing cancerous tissue located in a specific area, such as the breast, colon or skin, but is hardly used to treat cancerous tissue in some areas such as the spine. In addition, in the case of systemic chemotherapy that is commonly used for breast cancer, lung cancer, and testicular cancer, side effects may occur which disrupt normal cell replication or metabolic processes, and resistance to therapeutic agents used in chemotherapy may occur in patients.

Meanwhile, when cancer occurs in an individual, blood vessel formation and cell growth proceed at a very fast rate in the body, and hence an oxygen-deficient environment may be created in the cancer tissue due to incomplete blood vessel formation, and the cancer tissue may be very suitable for growth of anaerobic bacteria such as *Salmonella* sp. strains or *Escherichia coli.* Accordingly, current cancer therapies that use bacteria capable of targeting cancer, such as *Salmonella* sp. strains and *Clostridium* sp. strains, rely upon the functions of specific bacteria that can target solid tumors and proliferate within the tumors. However, when an oncolytic protein or a reporter protein is introduced into the bacteria and the transformed bacteria are administered to an individual, it is possible to specifically identify cancer tissue or to treat cancer by minimizing side effects that are toxic to normal cells.

Diseases are caused in humans by toxins secreted from various bacterial pathogens that exist in nature. Among various bacterial pathogens that can cause diseases, *Salmonella enterica,* etc., which are closely related to our diet, are known as Enterobacteriaceae that inhabit the intestinal tracts of primates, including humans, and secrete cytolysin, which is known as an exotoxin. Cytolysin that is secreted in this way is a cytotoxic protein having a molecular weight of about 34kDa, and is known to cause hemolysis (the destruction of red blood cells) in the intestines of primates, including humans, and to form pores in the membrane of normal cells to induce cell lysis, which leads to death due to severe vascular inflammation and the necrosis of local tissue. However, recent research results indicate that the cytolysin isolated and purified from *Salmonella enterica* acts specifically on cancer cells present in the intestinal tract, thereby inducing the death of the cancer cells. Thus, the cytolysin has attracted attention as a nextgeneration anticancer therapeutic agent. Therefore, bacteria transformed with a gene secreting the cytotoxic substance cytolysin may have a very high potential for use as an anticancer therapeutic agent for targeting cancer tissue.

Although it is possible to diagnose or treat cancer using bacteria as described above, there have been few studies on expression vectors that allow proteins suitable for diagnosis and treatment to be expressed specifically in cancer tissues. After the bacteria are injected into a living body, clearance occurs in the reticuloendothelial systems such as the liver and spleen for the first 3 days, and then the bacteria increase rapidly in cancer tissue after a certain period of time. Thus, in terms of safety, it is required to allow the expression of a therapeutic protein after a certain period of time. Accordingly, the use of an inducible promoter for the expression of a therapeutic protein is recommended, but the clinical application of inducible promoters, such as a P_{BAD} promoter currently used in the experimental stage, is greatly limited because L-arabinose, which is not permitted for human use, must be used as an inducer. A Pₜₑₜ promoter, which uses doxycycline, an antibiotic approved for human use, has advantages in that it is relatively easy to use clinically and allows bidirectional transcription of two genes using a TetA promoter and a TetR promoter. However, the difference in protein expression level between the TetA promoter and the TetR promoter is 100:1 or more, and thus the expression levels of these promoters need to be balanced to increase the utilization of the Pₜₑₜ promoter. Accordingly, it is necessary to develop a new technology for bacteria having a clinically applicable expression system and transformed such that protein expression levels can be balanced.

### DISCLOSURE

### Technical Problem

An object of the present disclosure is to provide a DNA construct.

Another object of the present disclosure is to provide a recombinant vector comprising the DNA construct.

Still another object of the present disclosure is to provide a strain into which the recombinant vector has been introduced, and a composition for diagnosing cancer comprising the strain.

Yet another object of the present disclosure is to provide a pharmaceutical composition for preventing or treating cancer containing the strain as an active ingredient.

Still yet another object of the present disclosure is to provide a method for providing information for diagnosing cancer, the method comprising a step of treating with the strain.

However, the technical problems to be solved by the present disclosure are not limited to the above-mentioned problems, and other problems not mentioned herein will be clearly understood by those skilled in the art from the following description.

### Technical Solution

An embodiment of the present disclosure provides a DNA construct.

The DNA construct of the present disclosure comprises genes encoding the first protein and the second protein, and a first promoter and a second promoter corresponding thereto, wherein the first protein is a flagellin and the second protein is a toxin protein.

The flagellin of the present disclosure is flagellin A or B.

The DNA construct of the present disclosure further comprises a gene encoding a regulatory protein.

According to another aspect of the present disclosure, the present disclosure comprises a recombinant vector individually comprising a DNA construct comprising a gene encoding a first protein and a first promoter corresponding thereto, and a DNA construct comprising a gene encoding a second protein and a second promoter corresponding thereto, and provides a strain into which the recombinant vector has been introduced, wherein the first protein is flagellin and the second protein is a toxin protein.

Since the first promoter and second promoter of the present disclosure are simultaneously inducible by a single regulatory protein expressed by a separate promoter, the expression levels of proteins encoded by the genes, operably linked downstream of the first promoter and the second promoter, in a host strain or cell, may be balance with each other, unlike the case in which the gene encoding the regulatory protein is operably linked downstream of the second promoter. Accordingly, when the DNA construct according to the present invention is used, diagnosis and treatment can be performed simultaneously.

The term "DNA construct" as used in the present disclosure refers to a construct that enables expression of a desired protein or the like when introduced into a host strain or cell by transformation, and comprises not only a gene encoding the desired protein, but also a nucleotide sequences corresponding to promoters, which are essential regulatory elements operably linked so that the gene can be expressed.

The term "promoter" as used in the present disclosure refers to a nucleotide sequence which is present upstream of an operably linked gene in a host strain or cell, and is the nucleotide sequence of a specific region of the DNA construct to which RNA polymerase may bind in order to initiate transcription.

Expression of the regulatory protein of the present disclosure may be regulated by a cis-acting element (cis-regulatory elements; CRE) or trans-acting factor (trans-regulatory elements; TRE).

The terms "regulation" to "regulation of expression" as used in the present disclosure may mean that transcription and translation of a specific gene are activated or inhibited.

The cis-acting factor of the present disclosure is a region of non-coding DNA that regulates the transcription of a neighboring gene, is an essential component of the gene regulatory network, and controls gene expression. The cis-acting factor may be at least one selected from the group consisting of a ribosome binding site (RBS), a 5'-untranslated region (5'-UTR), a transcription factor binding site and terminators, but is not limited thereto.

In the present disclosure, the ribosome binding site (RBS) is also referred to as the Shine-Dalgarno sequence (SD sequence). After the genetic information contained in DNA is transcribed into messenger RNA (mRNA), a ribosome must bind to this mRNA for translation to occur. At this time, the ribosome binding site refers to a short sequence that is present on the mRNA so that the ribosome can bind effectively to the mRNA.

In the present disclosure, the 5'-untranslated region (5'-UTR) refers to untranslated regions flanking both sides of a 5' coding region which is translated into amino acids of mRNA. It is considered junk in the evolutionary process, but is known to play a major role in regulating gene expression.

In the present disclosure, the transcription factor binding site is a DNA region that serves to turn on or off a specific gene nearby. The transcription factor binding site may be at least one selected from the group consisting of a promoter, an enhancer, and a silencer of the gene encoding the regulatory protein, but is not limited thereto.

The promoter of the gene encoding the regulatory protein of the present disclosure may include any promoter whose activity can be induced in the environmental conditions and developmental conditions of most host strains or cells. Preferably, the promoter may be a weak promoter.

The "weak promoter" of the present invention is a promoter that induces a transcript, transcribed from the gene operably linked downstream thereof, to be expressed at a level of 1 × 10⁻² or less, preferably 1 × 10⁻³ or less, and may include any promoter that allows the transcript to be expressed at a level of 1 × 10⁻³ or less as described above. For example, the weak promoter may be at least one selected from the group consisting of an *E. coli* σ70 promoter, an *E. coli* σS promoter, an *E. coli* σ32 promoter, a *B. subtilis* σA promoter, a *B. subtilis* σB promoter, the *Salmonella-derived* promoter K112706 or K112707, a bacteriophage T7 promoter, a bacteriophage SP6 promoter, a yeast-derived promoter, the eukaryotic promoter 1712004 or K076017, and a plant-derived promoter, but is not limited thereto.

The *E. coli* σ70 promoter of the present disclosure may be at least one selected from the group consisting of 114018, 114033, I14034, I732021, I742126, J01006, J23103, J23109, J23112, J23113, J23117, J23119, J23150, J23151, J44002, J48104, J56015, J64951, K088007, K119000, K119001, K1330002, K137029, K137030, K137031, K137032, K137085, K137086, K137087, K137088, K137089, K137090, K137091, K1585100, K1585101, K1585102, K1585103, K1585104, K1585105, K1585106, K1585110, K1585113, K1585115, K1585116, K1585117, K1585118, K1585119, K2486171, K256002, K256018, K256020, K256033, K292000, K823007, K823010, K823013, M13101, M13102, M13103, M13104, M13105, M13106, M13108, M13110, M31519, R1074, R1075 and S03331, but is not limited thereto.

The *E. coli* σS promoter of the present disclosure may be J45992 or J45993, but is not limited thereto.

The *E. coli* σ32 promoter of the present disclosure may be J45504, K1895002 or K1895003, but is not limited thereto.

The *B. subtilis* σA promoter of the present disclosure may be at least one selected from the group consisting of K143012, K143013, K823000, K823002 and K823003, but is not limited thereto.

The *B. subtilis* σB promoter of the present disclosure may be K143010, K143011 or K143013, but is not limited thereto.

The bacteriophage T7 promoter of the present disclosure may be at least one selected from the group consisting of I719005, J34814, J64997, K113010, K113011, K113012, K1614000, R0085, R0180, R0181, R0182, R0183, Z0251, Z0252 and Z0253, but is not limited thereto.

The bacteriophage SP6 promoter of the present disclosure may be J64998, but is not limited thereto.

The yeast-derived promoter of the present disclosure may be at least one selected from the group consisting of I766557, J63005, K105027, K105028, K105029, K105030, K105031, K122000, K124000, K124002, K319005, M31201, K2365040, K2365036, K2365041, K2365042, K2365032, K2365051, K2365514, K2365515 and K2365516, but is not limited thereto.

The plant-derived promoter of the present disclosure may be at least one selected from the group consisting of PLPR0203, PLPR0210, PLPR0177, PLPR0193, PLPR0507, PLPR0422, PLPR0228, PLPR0226, PLPR0223, PLPR0040, PLPR0465, PLPR0232, PLPR0205, PLPR0247, PLPR0328, PLPR0525, AtREG383, AtREG415, AtREG416, OsREG438, OsREG443, OsREG501, PpREG186, PpREG194 and PpREG197, but is not limited thereto.

For the purposes of the present disclosure, when the gene encoding the regulatory protein is operably linked downstream of the weak promoter, it is possible to control transcription such that transcription of the gene present downstream of the first and second promoters may occur specifically even when a substance that inhibits the regulatory protein is administered, unlike the case where the gene is operably linked downstream of the first promoter or the second promoter.

The promoter of the gene encoding the regulatory protein of the present disclosure may have the nucleotide sequence of SEQ ID NO:8 corresponding to the -35 site from the gene encoding the regulatory protein, and the nucleotide sequence of SEQ ID NO:9 corresponding to the -10 site, but is not limited thereto.

The enhancer of the present disclosure is a sequence found in both prokaryotes and eukaryotes, and generally has a 50 to 1,500bp region, is located upstream or downstream of the starting point of the nearby gene, and induces binding of the transcription factor.

The silencer of the present disclosure has the same mechanism as the enhancer and antagonizes the enhancer effect. The transcription factor that binds to the silencer is a repressor. The enhancer and the silencer may be present in regions adjacent to each other, or may be present in the same region in which the transcription factors thereof are different.

The terminators of the present disclosure are also referred to as transcription terminators, and mediate the termination of transcription of genes or operons in the genome. Prokaryotes include Rho-dependent terminators and Rho-independent terminators.

The trans-acting factor of the present disclosure is also referred to as a trans-activator or a trans-acting transcription factor, and is a factor that trans-activates the transcription of a gene. The trans-acting factor may be at least one selected from the group consisting of the transcription factor, an aptamer, sRNA, and antisense RNA (asRNA), but is not limited thereto.

In the present disclosure, the transcription factor is ae protein that helps turn on or off a specific gene by binding to the transcription factor binding site.

In the present disclosure, the aptamer is a part of a riboswitch, and collectively refers to oligonucleotide or peptide substances capable of binding to a specific target molecule. The aptamer is a peptide aptamer or a nucleic acid aptamer. The riboswitch is a kind of mRNA that regulates the expression of genes, and examples thereof include, but are not limited to, a glmS riboswitch, an RMN riboswitch, a Cobalamin riboswitch, and the like.

In the present disclosure, the sRNA or the antisense RNA (asRNA) refers to a single-stranded RNA capable of complementarily binding to a specific RNA. The antisense RNA binds complementarily to sense RNA, a messenger RNA (mRNA) expressing a certain protein, thereby regulating the expression of the protein.

The first promoter and the second promoter of the present invention may be inducible promoters that are induced by the regulatory protein.

The term "inducible promoter" as used in the present disclosure is a promoter that specifically transcribes the gene linked downstream thereof so that the gene can be expressed only under specific chemical or physical conditions. For example, the inducible promoter may be the LacZ gene promoter, which is expressed in the presence of galactose such as isopropyl-β-D-1-thiogalactopyranoside (IPTG), the arabinose operon araBAD promoter, which is expressed only in the presence of L-arabinose, or the tet promoter, whose expression is regulated by tetracycline. Preferably, the first promoter and the second promoter may be tet promoters. More preferably, the first promoter may be a tetA promoter, and the second promoter may be a tetR promoter, but the present disclosure is not limited thereto.

The gene encoding the regulatory protein of the present disclosure may be a protein that binds to the first promoter and the second promoter so that RNA polymerase cannot bind thereto. For the purposes of the present disclosure, when the first promoter and the second promoter are tet promoters, the gene may be the TetR protein capable of inhibiting the activity of each tet promoter by binding to the regulatory region of each tet promoter, but is not limited thereto.

The term "operably linked" as used in the present disclosure means that one nucleic acid fragment of interest is functionally linked to another nucleic acid fragment so that the function or expression thereof is affected by the other nucleic acid fragment.

The term "reporter protein" as used in the present disclosure refers to a protein that functions so that cancer can be visually diagnosed. For example, the reporter protein may be, but is not limited to, at least one selected from the group consisting of a fluorescent protein, luciferase, and a protein that is used in nuclear medicine or MRI imaging.

The term "fluorescent protein" as used in the present disclosure is a protein that fluoresces by itself so that cancer can be visually diagnosed. For example, the fluorescent protein may be at least one selected from the group consisting of green fluorescent protein (GFP), modified green fluorescent protein (MGFP), enhanced green fluorescent protein (EGFP), red fluorescent protein (RFP), enhanced red fluorescent protein (ERFP), blue fluorescent protein (BFP), enhanced blue fluorescent protein (EBFP), yellow fluorescent protein (YFP), and enhanced yellow fluorescent protein (EYFP), but is not limited thereto.

In the present disclosure, the protein that is used in nuclear medicine or MRI imaging may be, for example, at least one selected from the group consisting of herpes simplex virus thymidine kinase, dopamine receptor, somatostatin receptor, sodium-iodide transporter, iron receptor, transferrin receptor, ferritin and iron transporter (magA), but is not limited thereto.

The term "cytokine" as used in the present disclosure refers to proteins secreted by immune cells, and the cytokine of the present disclosure may include any cytokine that may be used in cancer immunotherapy capable of inducing the death of disease-related cells, for example, cancer cells, by regulating host immune response. Preferably, examples of the cytokine include, but are not limited to, IFN-α2, IL-2, IL-15, IL-21 and IL-12.

The term "chemokine" as used in the present disclosure refers to one functioning to control the migration of cells between tissues and the positioning and interactions of cells within tissues. The chemokine may include any chemokine that may mediate the host response to diseases, for example, cancer, by directing the trafficking of leukocytes into the tumor microenvironment. Preferably, examples of the chemokine include, but are not limited to, CXCR3, CCR5, etc.

The term "immune modulator" as used in the present disclosure refers to a modulator which enables various treatments by utilizing the intrinsic immune system of an individual, and may be any modulator that can induce the death of disease-related cells, for example, cancer cells, by activating immune cells.

The "anticancer protein" of the present disclosure refers to a peptide having a function capable of directly or indirectly inducing the death of cancer cells. The anticancer protein may be, for example, at least one selected from the group consisting of a toxin protein, an antibody specific for a cancer antigen or a fragment of the antibody, a tumor suppressor protein, an angiogenesis inhibitor, a cancer antigen, a prodrug-converting enzyme, and a pro-apoptotic protein, but is not limited thereto.

The term "toxin protein" as used in the present disclosure refers to a protein having a function capable of directly or indirectly inducing the death of cancer cells. For example, the toxin protein may be at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), streptolysin O (SLO), pneumolysin (PLO) listeriolysin (LLO), and cytolysin A (ClyA). More preferably, the toxin protein may be cytolysin A consisting of the amino acid sequence represented by SEQ ID NO: 1, but is not limited thereto.

The term "tumor suppressor protein" as used in the present disclosure refers to a gene which maintains its function in normal cells, but causes normal cells to indiscriminately divide and grow into cancer cells when the function thereof is lost. Examples of the tumor suppressor protein include, but are not limited to, retinoblastoma (RB) protein, p53 protein, adenomatous polyposis coli (APC) protein, phosphatase and tensin homologue (PTEN) protein, cyclin dependent kinase inhibitor 2A (CDKN2A) protein, and the like.

In the present disclosure, the antibody specific for cancer antigen and the fragment of the antibody is an antibody capable of specifically binding to an antigen which is a protein having a high expression level specifically on the surface or cytoplasm of a cancer cell. For example, the antibody or the fragment thereof may be an antibody specific for HER2 having a high expression level specifically in breast cancer or gastric cancer cells, but is not limited thereto.

The term "antibody" as used in the present disclosure refers to a protein molecule capable of binding specifically to an antigenic site of a protein or peptide molecule. The type of the antibody is not particularly limited, and examples thereof include polyclonal antibodies, monoclonal antibodies, or antibody fragments having an antigen-binding property, and include all types of immunoglobulin antibodies. In addition, examples of the antibody include specific antibodies such as humanized antibodies. Examples of the antibody include not only a whole antibody having two full-length light chains and two full-length heavy chains, but also a functional fragment of an antibody molecule. The "functional fragment of an antibody molecule" refers to a fragment having at least an antigen-binding function, and examples thereof include, but are not limited, Fab, F(ab'), F(ab')2, Fv, etc.

The term "antibody" of the present disclosure may be produced by a conventional method after cloning the gene encoding the cancer antigen of the present disclosure into an expression vector according to a conventional method to obtain a protein encoded by the gene.

The term "angiogenesis inhibitor" as used in the present disclosure refers to a protein or compound having a function capable of directly or indirectly inducing the death of cancer cells by inhibiting the formation of new blood vessels around cancer cells. Preferably, examples of the angiogenesis inhibitor include, but are not limited to, angiostatin, endostatin, thrombospondin, and protease inhibitory proteins.

The term "cancer antigen" as used in the present disclosure refers to a protein antigen which is expressed in cancer cells but is rarely expressed in normal cells, thereby inducing an anti-tumor immune response, thereby inducing the direct or indirect death of cancer cells. Preferably, the cancer antigen of the present disclosure may be α-fetoprotein (AFP), vascular endothelial growth factor receptor 2 (VEGFR2), Survivin, Legumain, prostate cancer-specific antigen (PCSA), or the like, but is not limited thereto.

The term "prodrug converting enzyme" as used in the present disclosure refers to a protein having a function of converting an inactive drug into an active drug through a metabolism caused by an enzymatic reaction. When this prodrug converting enzyme is used, the inactive drug can be metabolized and converted into an active drug capable of directly or indirectly inducing the death of cancer cells. Thus, the prodrug converting enzyme may be very useful for the prevention or treatment of cancer. Preferred examples of the prodrug converting enzyme of the present disclosure include, but are not limited to, thymidine kinase, cytosine deaminase, nitroreductase, purine nucleoside phosphorylase, carboxypeptidase G2, chromate reductase YieF, herpes simplex virus type I thymidine kinase/ganciclovir (HSV1-TK/GCV), β-glucuronidase, and the like.

The term "pro-apoptotic protein" as used in the present disclosure refers to a protein that induces the direct or indirect death of cancer cells by making these cells deficient in factors (proteins, nutrients, oligonucleotides, etc.) essential for growth or maintenance of the cancer cells. Preferred examples of the pro-apoptotic protein of the present disclosure include, but are not limited to, L-ASNase, RNA-binding motif protein 5 (RBM5), and the like.

The cancer antigen-specific oligonucleotide of the present disclosure refers to a nucleotide capable of inhibiting the expression or function of a cancer antigen by complementary binding to a gene or mRNA of the cancer antigen, and may be any one selected from the group consisting of an antisense oligonucleotide, an aptamer, siRNA and shRNA, but is not limited thereto.

The term "antisense oligonucleotide" as used in the present disclosure refers to DNA, RNA, or a derivative thereof, which comprises a nucleic acid sequence complementary to a specific mRNA sequence and is capable of inhibiting the translation of mRNA into protein by binding to the complementary sequences in mRNA. The antisense oligonucleotide may be synthesized *in vitro* by a conventional method using, for example, RNA polymerase I, and then administered *in vivo,* or may be synthesized *in vivo* by a method using a vector having a multiple cloning site (MCS) in opposite orientation.

The term "aptamer" as used in the present disclosure refers to a small single-stranded oligonucleotide capable of specifically recognizing a target substance with high affinity. For the purposes of the present disclosure, the target substance may be a gene or mRNA of a cancer antigen.

The term "siRNA" as used in the present disclosure refers to a short double-stranded RNA capable of inducing an RNA interference (RNAi) phenomenon by cleavage of a specific mRNA. The siRNA is composed of a sense RNA strand having a sequence homologous to the mRNA of a target gene and an antisense RNA strand having a sequence complementary thereto. For the purposes of the present disclosure, the siRNA may bind specifically to an mRNA transcribed from a gene encoding a cancer antigen, thereby effectively inhibiting the expression of this gene.

The term "shRNA" as used in the present disclosure refers to a short hairpin RNA. The shRNA has advantages in that the cell transfection rate thereof is higher than that of siRNA and that RNA interference can be maintained for a long period of time. RNA interference can be induced by, but not limited to, the process of transforming adenovirus, lentivirus and plasmid expression vector systems from the promoter of RNA polymerase III into cells, followed by expression. For the purposes of the present disclosure, the shRNA may specifically bind to mRNA transcribed from a gene encoding a cancer antigen, thereby effectively inhibiting the expression of this gene.

Another embodiment of the present disclosure provides a recombinant vector comprising the DNA construct of the present disclosure.

As the recombinant vector of the present disclosure comprises the DNA construct of the present disclosure, the regulatory protein is expressed by a separate promoter, and thus the genes operably linked downstream of the first promoter and the second promoter may be expressed in a balanced manner, specifically only when a substance that inhibits the regulatory protein is externally administered.

In the recombinant vector of the present disclosure, details regarding the DNA construct, anticancer protein, cytokine, chemokine, immune modulator, cancer antigen-specific oligonucleotide, reporter protein and promoter, etc. are the same as described above with respect to the DNA construct, and thus the repeated description thereof will be omitted in order to avoid excessive complexity of the present specification.

The recombinant vector of the present disclosure is a means for expressing a protein by introduction into a cell, and may be a known recombinant vector such as a plasmid vector, a cosmid vector or a bacteriophage vector. The recombinant vector may be readily produced by those skilled in the art according to any known method using DNA recombination technology. In the present disclosure, specific examples of the recombinant vector may be selected from the group consisting of a pCDNA vector which is commercially widely used, F, R1, RP1, Col, pBR322, ToL, Ti vector, cosmid, phages such as lambda, lambdoid, M13, Mu, p1 P22, Qµ, T-even, T2, T3, or T7, and plant viruses, but are not limited thereto. For the purposes of the present disclosure, a suitable recombinant vector may be selected according to the nature of the host cell.

Another embodiment of the present disclosure provides a host cell or strain into which a recombinant vector comprising the DNA construct of the present disclosure has been introduced.

The host cell of the present disclosure may include cells of mammalian, plant, insect, fungal or cellular origin. For example, the host cell may be, but is not limited to, at least one selected from the group consisting of bacterial cells such as *Escherichia coli, Streptomyces* or *Salmonella* sp. strains; fungal cells such as yeast cells or *Pichia pastoris*; insect cells such as Drosophila or Spodoptera Sf9 cells; animal cells such as Chinese hamster ovary (CHO) cells, SP2/0 (mouse myeloma), human lymphoblastoid, COS, NSO (mouse myeloma), 293T cells, bow melanoma cells, HT-1080 cells, baby hamster kidney (BHK) cells, human embryonic kidney (HEK) cells, or PERC.6 cells (human retinal cells); and plant cells. For the purposes of the present disclosure, the strain may be at least one selected from the group consisting of anaerobic strains, for example, *Salmonella* sp. strains, *Clostridium* sp. strains, *Bifidobacterium* sp. strains, and *E*. *coli* strains. Preferably, the strain may be at least one selected from the group consisting of *Salmonella typhimurium*, *Salmonella choleraesuis* and *Salmonella enteritidis.* More preferably, the strain may be *Salmonella typhimurium*, but is not limited thereto.

The strain of the present disclosure may be an attenuated strain.

The term "attenuated" as used in the present disclosure means modifying a gene or the like so as to reduce toxicity and other side effects, which may occur when a microorganism is administered to a patient. For the purposes of the present disclosure, when the strain is a *Salmonella* sp. strain, at least one gene selected from the group consisting of *aroA*, *aroC*, *aroD*, *aroE*, *Rpur*, *htrA*, *ompR*, *ompF*, *ompC*, *galE*, *cya*, *crp*, *cyp*, *phoP*, *phoQ*, *rfaY*, *dksA, hupA, sipC*, *clpB, clpP, clpX*, *pab*, *nada*, *pncB*, *pmi*, *rpsL*, *hemA*, *rfc*, *poxA*, *galU*, *cdt*, *pur*, *ssa*, *guaA*, *guaB*, *fliD,flgK*,*flgL*, *relA* and *spoA* may be modified for attenuation, but is not limited thereto.

The method for modifying the gene of the present disclosure may be performed by a method of deleting or disrupting various genes as known in the art. For example, the deletion or disruption method may be performed by a method such as homologous recombination, chemical mutagenesis, irradiation mutagenesis or transposon mutagenesis.

In the present disclosure, the strain targets the inside of cancer tissue, which is an oxygen-deficient environment which is very suitable for the growth of an anaerobic strain and shows incomplete blood vessel formation, and thus when a recombinant vector comprising a reporter protein that may be imaged in real time and an anticancer protein is introduced into this strain so that the expression levels of the reporter protein and the anticancer protein can be balanced, it is possible to very effectively diagnose and treat cancer.

In the strain of the present disclosure, details regarding the DNA construct, anticancer protein, cytokine, chemokine, immune modulator, cancer antigen-specific oligonucleotide, reporter protein, promoter and recombinant vector, etc., are the same as described above with respect to the DNA construct and the recombinant vector, and thus the repeated description thereof will be omitted in order to avoid excessive complexity of the present specification.

The recombinant vector of the present disclosure may be introduced into a host cell or a strain by transformation (or transfection), and the transformation method that is used in the present disclosure may be any transformation method and may be easily performed according to a conventional method known in the art. Specifically, the recombinant vector may be introduced into the strain by a method for transformation of bacteria such as the *Salmonella* sp. strain, which may be commonly used, CaCl₂ precipitation, Hanahan method that uses DMSO (dimethyl sulfoxide) as a reducing material in addition to the CaCl₂ method to increase efficiency, electroporation, calcium phosphate precipitation, protoplast fusion, an agitation method using silicon carbide fibers, Agrobacterium mediated transformation, transformation using PEG, a method using dextran sulfate, a method using lipofectamine, or desiccation/inhibition-mediated transformation, but the transformation method is not limited thereto.

Another embodiment of the present disclosure provides a pharmaceutical composition for preventing or treating cancer.

According to another aspect of the present disclosure, the present disclosure provides a pharmaceutical composition for preventing or treating cancer comprising the strain of the present disclosure as an active ingredient.

The pharmaceutical composition of the present disclosure inhibits cancer growth or cancer metastasis.

As the strain of the present disclosure is transformed with the DNA construct of the present disclosure, it may target cancer in an individual, and then the reporter protein that may be imaged in real time and the anticancer protein in this strain may be expressed in a balanced manner when a substance that inhibits the regulatory protein is administered. Thus, the strain may very effectively prevent or treat cancer, and at the same time, may diagnose cancer in real time.

The term "cancer" as used in the present disclosure refers to a disease characterized by rapid and uncontrolled growth of mutant cells. The cancer may be at least one selected from the group consisting of melanoma, fallopian tube cancer, brain cancer, small intestine cancer, esophageal cancer, lymph adenocarcinoma, gallbladder cancer, blood cancer, thyroid cancer, endocrine adenocarcinoma, oral cancer, liver cancer, biliary tract cancer, colorectal cancer, rectal cancer, cervical cancer, ovarian cancer, kidney cancer, stomach cancer, duodenal cancer, prostate cancer, breast cancer, brain tumor, lung cancer, undifferentiated thyroid cancer, uterine cancer, colon cancer, bladder cancer, ureter cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myeloid leukemia, chronic lymphocytic leukemia, chronic myelogenous leukemia, and solitary myeloma. Preferably, the cancer may be at least one selected from the group consisting of liver cancer, biliary tract cancer, colorectal cancer, rectal cancer, cervical cancer, ovarian cancer, kidney cancer, stomach cancer, duodenal cancer, prostate cancer, breast cancer, brain tumor, lung cancer, undifferentiated thyroid cancer, uterine cancer, colon cancer, bladder cancer, ureter cancer, pancreatic cancer, bone/soft tissue sarcoma, and skin cancer. More preferably, the cancer may be colon cancer, but is not limited thereto.

The term "prevention" as used in the present disclosure may include, without limitation, any action that blocks symptoms caused by cancer or suppresses or delays the symptoms, by using the active ingredient of the present disclosure.

The term "treatment" as used in the present disclosure refers to any action that beneficially changes symptoms caused by cancer or benefits an individual, by using the active ingredient of the present disclosure, and refers to an approach for obtaining beneficial or desired results, including clinical results. Beneficial or desired clinical results may include, but are not limited to, alleviation or amelioration of one or more symptoms or conditions, diminishment of extent of disease, stabilization of the state of disease, prevention of development of disease, prevention of spread of disease, delay or slowing of disease progression, delay or slowing of disease onset, amelioration or palliation of the disease state, and remission (whether partial or total), whether detectable or undetectable. "Treatment" may also mean prolonging survival of a patient beyond that expected in the absence of treatment. In addition, "treatment" can also mean inhibiting the progression of disease or slowing the progression of disease temporarily, although more preferably, it involves halting the progression of the disease permanently. As will be understood by a skilled person, results may not be beneficial or desirable if, while improving a specific disease state, the treatment results in adverse effects on the patient treated that outweigh any benefits effected by the treatment.

In the present disclosure, the treatment may be suppression of cancer metastasis or cancer recurrence.

In the present disclosure, "cancer metastasis" refers to the characteristic of cancer moving to another place separated by a distance from a primary organ or part. Metastatic cancer is a cancer that tends to metastasize or has been metastasized. Particularly, the metastatic cancer may be metastasized to the liver, lung, bone, lymph node or abdominal cavity, but is not limited thereto. Metastatic cancer is difficult to treat, and the progression and treatment may be more complicated than the initial treatment process.

In the present disclosure, "cancer recurrence" means that cancer that was not found after treatment is found again after a certain period of time. Recurrent cancer refers to cancer resulting from recurrence of cancer as described above. When cancer recurs, resection is often difficult, and even if resection is possible, major surgery may be required. In addition, there may be limitations to chemotherapy and radiation therapy.

In the pharmaceutical composition of the present disclosure, details regarding the DNA construct, anticancer protein, cytokine, chemokine, immune modulator, cancer antigen-specific oligonucleotide, reporter protein, promoter recombinant vector, strain and transformation, etc. are the same as described above with respect to the DNA construct, the recombinant vector and the strain, and thus the repeated description thereof will be omitted in order to avoid excessive complexity of the present specification.

The pharmaceutical composition of the present disclosure may be in the form of capsules, tablets, granules, injections, ointments, powders or beverages, and the pharmaceutical composition may be for administration to humans.

For use, the pharmaceutical composition of the present disclosure may be formulated in the form of, but not limited to, oral dosage forms such as powders, granules, capsules, tablets, aqueous suspensions, etc., external preparations, suppositories, and sterile injection solutions, according to the respective conventional methods. The pharmaceutical composition of the present disclosure may contain pharmaceutically acceptable carriers. Pharmaceutically acceptable carriers that may be used for oral administration include binders, lubricants, disintegrants, excipients, solubilizers, dispersants, stabilizers, suspending agents, pigments, flavorings, and the like, and pharmaceutically acceptable carriers that may be used for injection include buffers, preservatives, analgesics, solubilizers, isotonic agents, stabilizers, and the like. Pharmaceutically acceptable carriers that may be used for topical administration include bases, excipients, lubricants, preservatives, and the like. The dosage forms of the pharmaceutical composition of the present disclosure may be prepared in various ways by mixing with pharmaceutically acceptable carriers as described above. For example, for oral administration, the pharmaceutical composition may be prepared in the form of tablets, troches, capsules, elixir, suspensions, syrups, wafers, and for injection, the pharmaceutical composition may be presented in unit dose ampoules or multi-dose containers. In addition, the pharmaceutical composition may be formulated as solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition may further contain a filler, an anticoagulant, a lubricant, a wetting agent, a fragrance, an emulsifier, a preservative, or the like.

The routes of administration of the pharmaceutical composition according to the present disclosure include, but are not limited to, oral, intravenous, intramuscular, intra-arterial, intramedullary, intradural, intracardiac, transdermal, subcutaneous, intraperitoneal, intranasal, gastrointestinal, topical, sublingual and intrarectal routes. Oral or parenteral administration is preferred.

In the present disclosure, "parenteral" includes subcutaneous, transdermal, intravenous, intramuscular, intra-articular, intra-synovial, intrasternal, intradural, intra-lesional and intracranial injection or infusion techniques. The pharmaceutical composition of the present disclosure may also be formulated as suppositories for intrarectal administration.

The pharmaceutical composition of the present disclosure may vary depending on various factors, including the activity of a specific compound used, the patient's age, body weight, general health, sex, diet, the time of administration, the route of administration, excretion rate, the drug content, and the severity of a specific disease to be prevented or treated. The dose of the pharmaceutical composition may be suitably selected by a person skilled in the art depending on the patient's condition, body weight, the severity of the disease, the form of drug, and the route and period of administration, and may be 0.0001 to 50 mg/kg/day or 0.001 to 50 mg/kg/day. The pharmaceutical composition may be administered once or several times a day. The dose is not intended to limit the scope of the present disclosure in any way. The pharmaceutical composition according to the present disclosure may be formulated as pills, sugar-coated tablets, capsules, liquids, gels, syrups, slurries, or suspensions.

Another embodiment of the present disclosure provides a composition for diagnosing cancer.

The diagnostic composition of the present disclosure comprises the strain of the present disclosure as an active ingredient.

As the strain of the present disclosure is transformed with the DNA construct of the present disclosure, it may target cancer cells in an individual, and then the reporter protein that may be imaged in real time and the anticancer protein in this strain may be simultaneously expressed in a balanced manner when a substance that inhibits the regulatory protein is administered. Thus, the strain may very effectively prevent or treat cancer, and at the same time, may diagnose cancer in real time.

The term "diagnosing" as used in the present disclosure refers to any action that detects cancer tissue *in vivo,* including monitoring the presence of cancer in real time by the reporter protein expressed from the DNA construct introduced into the strain, when the strain of the present disclosure is located by targeting cancer.

In the diagnostic composition of the present disclosure, details regarding the DNA construct, anticancer protein, reporter protein, constitutive promoter, inducible promoter, recombinant vector, *Salmonella* sp. strain, transformation, cancer, etc., are the same as described above with respect to the DNA construct, the recombinant vector, the strain and the pharmaceutical compositions, and thus the repeated description thereof will be omitted in order to avoid excessive complexity of the present specification.

Another embodiment of the present disclosure provides a method for providing information for diagnosing cancer.

The method of the present disclosure comprises a step of treating a biological sample, isolated from a subject of interest, with the strain into which the recombinant vector according to the present disclosure has been introduced.

The method for providing information for diagnosing cancer according to the present disclosure may further comprise a step of diagnosing cancer when the reporter protein is expressed from the strain.

The term "biological sample" as used in the present disclosure refers to any material, tissue or cell obtained or derived from the subject. Examples of the biological sample include, but are not limited to, tissues, cells, or cell extracts.

In the method for providing information for diagnosing cancer according to the present disclosure, details regarding the DNA construct, anticancer protein, cytokine, chemokine, immune modulator, cancer antigen-specific oligonucleotide, reporter protein, promoter, recombinant vector, strain, transformation, cancer, diagnosis, etc. are the same as described above with respect to in the DNA construct, the recombinant vector, the strain, the pharmaceutical composition, and the diagnostic composition, and thus the repeated description thereof will be omitted in order to avoid excessive complexity of the present specification.

The flagellin of the present disclosure may include a DNA construct that is flagellin A or B, but is not limited thereto.

A strain used for preventing or treating cancer of the present invention include strains introduced with the recombinant vectors or flagellin and toxin proteins of the present invention.

A method of preventing or treating cancer by administering an effective amount of the strain introduced with the recombinant vector of the present disclosure or the flagellin and toxin protein recombinant vectors into to a target subject, is included.

Another embodiment of the present disclosure provides a method for preventing or treating cancer by administering effective amounts of an exogenous flagellin-expressing strain and an exogenous toxin protein-expressing strain in combination to a target subject.

The term "flagellin" as used in the present disclosure is, but is not limited to, a granular protein constituting the helical fibers of bacterial flagellum, and its molecular weight varies greatly depending on the species of bacteria (30,000~70,000). In terms of amino acid composition, cysteine and Tryptophan are absent, and in the case of *Salmonella*, about half of the lysines are methylated. Its types include flagellin A and B, and although their functions are diverse, they are known to have an immune enhancing effect.

In the present disclosure, "administration in combination" is a method of using diverse methods (surgery, radiation therapy, chemotherapy, immunotherapy, etc.) together or stepwise for cancer treatment. Although not limited thereto, two or more anticancer agents or drugs with different mechanisms are administered, or physical therapy such as radiation therapy and chemotherapy such as anticancer drugs are simultaneously used. When two drugs are used, synergistic effects are shown based on complementary mechanisms or adjustment of drug dosages. However, although the synergistic effect is significantly reduced in actual clinical practice, the risk of serious side effects such as immunosuppressive action or cardiotoxicity may also be caused.

According to another aspect of the present disclosure, the present disclosure provides a composition for preventing or treating cancer comprising flagellin or a nucleotide encoding the same, and a toxin protein or a nucleotide enoding the same as an active ingredient.

In the present disclosure, details regarding flagellin or toxin protein have already been described above, and thus the description thereof are omitted to avoid excessive redundancy.

The gene of the present disclosure may be delivered in a gene delivery system, but may also exert similar pharmacological effects by being administered in the form of a translated peptide.

### Advantageous Effects

The DNA construct according to the present disclosure can prevent and treat cancer by controlling the expression level of an anticancer gene operably linked downstream of the first promoter and the second promoter in a host strain or cell.

In addition, the DNA construct can enable the expression of anticancer proteins at dosages suitable for cancer treatment by controlling the treatment of doxycycline.

### Brief Description of Drawings

FIG.1 is a schematic view of a DNA construct according to Preparation Example 1 of the present disclosure.
FIG. 2 shows the results of analyzing the growth pattern of the strain recombined with the DNA construct according to Example 1 of the present disclosure.
FIG. 3 shows the results of the hemolytic activity of blood agar of the strain according to Example 1 of the present disclosure.
FIG. 4 shows the results of measuring TLR-5 signal activation according to Example 1 of the present disclosure.
FIG. 5 shows the difference in expression level of the recombinant strain according to the doxycycline concentration according to Example 1 of the present disclosure.
FIG. 6 shows the anticancer effect of the recombinant strain according to Example 2 of the present disclosure.
FIG. 7 shows the cancer-targeting effect of the recombinant strain according to Example 3 of the present disclosure.
FIG. 8 shows the cancer-targeting effect of the recombinant strain according to Example 3 of the present disclosure.
FIG. 9 shows the anticancer effect of the recombinant strain according to Example 4 of the present disclosure.
FIG. 10 shows the anticancer effect of the recombinant strain according to Example 4 of the present disclosure.
FIG. 11 shows an experimental method for confirming the anticancer effect of the recombinant strain according to the Example of the present disclosure.
FIG. 12 shows the anticancer effect of the recombinant strain according to Example 5 of the present disclosure.
FIG. 13 shows the anticancer effect of the recombinant strain according to Example 5 of the present disclosure.
FIG. 14 shows the anticancer effect of the recombinant strain according to Example 6 of the present disclosure.
FIG. 15 shows the anticancer effect of the recombinant strain according to Example 5 of the present disclosure.
FIG. 16 shows the cancer recurrence inhibitory effect of the recombinant strain according to Example 7 of the present disclosure.
FIG. 17 shows the cancer recurrence inhibitory effect of the recombinant strain according to Example 7 of the present disclosure.
FIG. 18 shows the cancer metastasis inhibitory effect of the recombinant strain according to Example 8 of the present disclosure.
FIG. 19 shows the cancer metastasis inhibitory effect of the recombinant strain according to Example 8 of the present disclosure.
FIG. 20 shows the anticancer effect of the recombinant strain according to Example 9 of the present disclosure.
FIG. 21 shows the anticancer effect of the recombinant strain according to Example 9 of the present disclosure.
FIG. 22 shows the anticancer effect of the recombinant strain according to Example 9 of the present disclosure.

### Mode for Invention

Hereinafter, the present disclosure will be described in more detail through examples. It will be obvious to those skilled in the art that these examples are only for explaining the present disclosure in more detail, and the scope of the present disclosure according to the subject matter of the present disclosure is not limited by these examples.

### [Preparation Example 1] Construction of DNA Constructs Controllable by Doxycycline

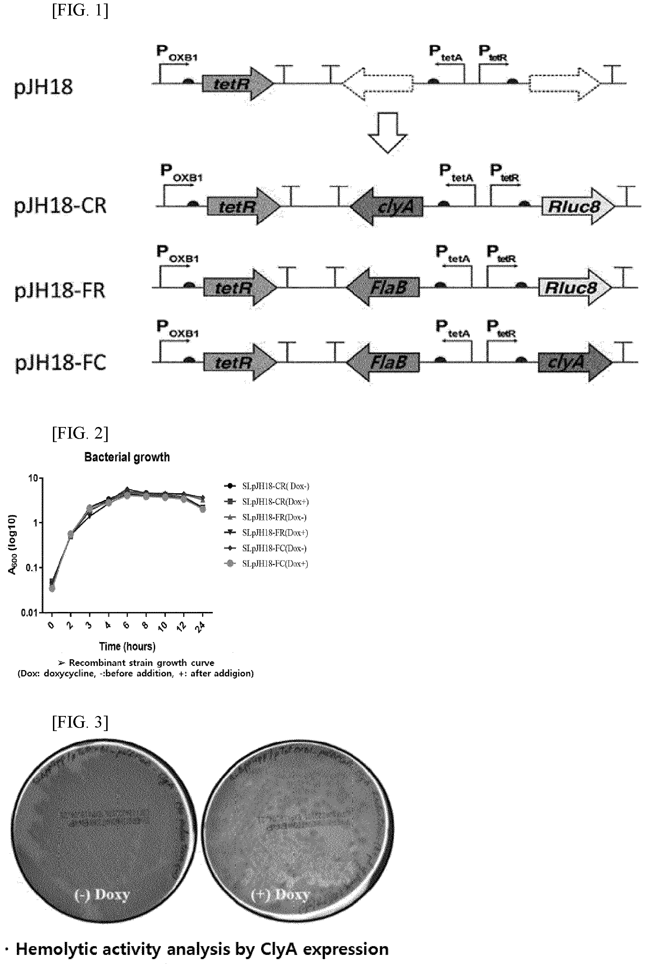

Using a pJL39 plasmid (Mol Ther., 21(11), p. 1985-1995, (2013)) as a template strand (FIG. 1), a tetR gene was amplified using a forward primer (5'-CGGAATTCACCATGTCTAGATTAGATAAAAGTAAAGTGATTAACAG-3'; SEQ ID NO: 2), constructed to include the restriction enzyme EcoRI site, and a reverse primer (5'-GCTCTAGACAGCTGTTAAGACCCACTTTCACATTTAAGTTGTTTTTCT-3'; SEQ ID NO: 3) constructed to include the restriction enzyme PvuII-XbaI site. Thereafter, the amplification product was cleaved with the restriction enzymes EcoRI and XbaI and purified to obtain a tetR gene amplification product which was then introduced into a pBAD24 (Catalog No. ATCC^{®} 87399^{™}, ATCC, USA) plasmid, thereby constructing a pBAD-TetR plasmid.

Thereafter, through PvuII and HindIII fragments of the pJL39 plasmid, a divergent promoter region containing a multiple cloning site was introduced into the pBAD-TetR plasmid, thereby constructing a pTetR-BAD plasmid. Using NheI and Pcil restriction enzymes, the araC and araBAD promoter were removed from the pTetR-BAD plasmid, thereby constructing a pTetII plasmid.

The constitutive promoter OXB1 (SEQ ID NO: 16), obtained by amplification using pSF-OXB1 (Oxford Genetics, England) as a template and a forward primer (5'-CTACTCCGTCAAGCCGTCAAGCTGTTGTGACCGCTTGCT-3'; SEQ ID NO: 4) and a reverse primer (5'-TGAATTCCTCCTGCTAGCTAGTTGGTAACGAATCAGACGCCGGGTAATACCGGAT AG-3'; SEQ ID NO: 5), was introduced into the pTetII plasmid by the Gibson assembly method, thereby constructing a pJH18 plasmid comprising the OXB 1, tetA and tetR promoters.

**[Table 1]**

| Plasmid | OXB1 | tetA | tetR |
|---|---|---|---|
| pJH18-CR | tetR | cly A | Rluc8 |
| pJH18-FR | tetR | FlaB | Rluc8 |
| pJH18-FC | tetR | FlaB | clyA |

### [Preparation Example 2] Cancer Cell Lines and Culture Conditions

The CT26 colon cancer cell lines CRL-2638 and HB-8064 (ATCC, USA) and the murine colorectal adenocarcinoma cell line MC38 (Massachusetts General Hospital and Harvard Medical School, USA and Chonnam National University, Korea) were used in the experiment.

Using high-glucose DMEM (Dulbecco's Modified Eagles Medium) (Catalog No. #LM 001-05, Welgene, Korea) containing 10% fetal bovine serum (FBS) and 1% penicillinstreptomycin, the cells were cultured in a 5% CO₂ incubator at 37°C.

### [Preparation Example 3] Construction of Salmonella Strains Having Plasmids Introduced Therein

As a *Salmonella* strain, SHJ2037 (relA::cat, spoT::kan), which is a ppGpp-deficient *Salmonella typhimurium (S. typhimurium)*, was used.

Each of the plasmids constructed in Preparation Example 1 above was transformed into the *Salmonella* strain by electroporation, and each of the transformed strains was cultured overnight in an LB containing 100 µg/ml ampicillin. Thereafter, each of the cultures was diluted at a ratio of 1:100 with a fresh LB medium containing ampicillin and further cultured. When the OD₆₀₀ value reached 0.5 to 0.7, doxycycline diluted with ethanol to a final concentration of 0, 10, 50, 100, 300 or 500 ng/ml was added to the cultures which were then cultured in a shaking incubator under conditions of 200 rpm and 37°C.

### [Preparation Example 4] Preparation of Experimental Animal Models

5 to 6-week-old C57BL/6 and BALB/C mice (Orient Company, Korea) weighing 20 to 30 g were used. MC38 or CT26 of Preparation Example 2 was subcutaneously injected into the flank of each of the mice, thereby constructing tumor animal models.

For imaging of the tumor animal model and evaluation of the tumor volume, 2% isoflurane was used for anesthesia, and 200 mg/kg of ketamine and 10 mg/kg of xylasine were used during surgery.

The tumor volume (mm³) was calculated using the equation (length × height × width)/2, and when the tumor volume in the animal model reached 1,500 mm³ or larger, the animal model was euthanized

### [Preparation Example 4] Preparation of Experimental Animal Models

5 to 6-week-old C57BL/6 and BALB/C mice (Orient Company, Korea) weighing 20 to 30 g were used. MC38 or CT26 of Preparation Example 2 was subcutaneously injected into the flank of each of the mice, thereby constructing tumor animal models.

For imaging of the tumor animal model and evaluation of the tumor volume, 2% isoflurane was used for anesthesia, and 200 mg/kg of ketamine and 10 mg/kg of xylasine were used during surgery.

The tumor volume (mm³) was calculated using the equation (length × height × width)/2, and when the tumor volume in the animal model reached 1,500 mm³ or larger, the animal model was euthanized

### [Example 1] Evaluation of Protein Expression and Activity of Recombinant Strain

### [1-1] Comparison of Growth of Recombinant and Conventional Strain

The recombinant strains SLpCR, SLpFR, and SLpFC prepared in Preparation Example 3, and SLpEmpty, used as a control, were grown overnight in LB liquid medium containing ampicillin, and then diluted at a ratio of 1:100 using fresh LB medium and further cultured. When the OD600 value reached 0.5 to 0.7, doxycycline diluted with ethanol to a final concentration of 200 ng/ml was added to the cultures which were then cultured in a shaking incubator under conditions of 200 rpm and 37°C. The growth pattern of the strain was analyzed by measuring the OD600 value according to the incubation time, and the results are shown in FIG. 2.

As shown in FIG. 2, the SLpCR, SLpFR and SLpFC recombinant strains showed little difference in growth rate compared to the control SLpEmpty strain, and did not show any particular growth inhibition even during protein expression using doxycycline. Therefore, it was confirmed that the lack of pathogenic genes in the strain prepared as described above did not affect the growth and gene expression of the strain.

### [1-2] Confirmation of Selective Hemolytic Activity of Recombinant Strain

After diluting the recombinant strain cultured in Preparation Example 3 with PBS, it was spread on a blood agar plate containing 0 or 20 ng/ml of doxycycline, incubated overnight at 37°C. A photograph of the plate was taken, and the results are shown in FIG. 3.

As shown in FIG. 3, the hemolytic activity of the strain's blood agar appeared only when doxycycline of the gene encoding cytolysin A was included (+).

### [1-3] Confirmation of TLR-5 Signaling Activation by FlaB in Recombinant Strain

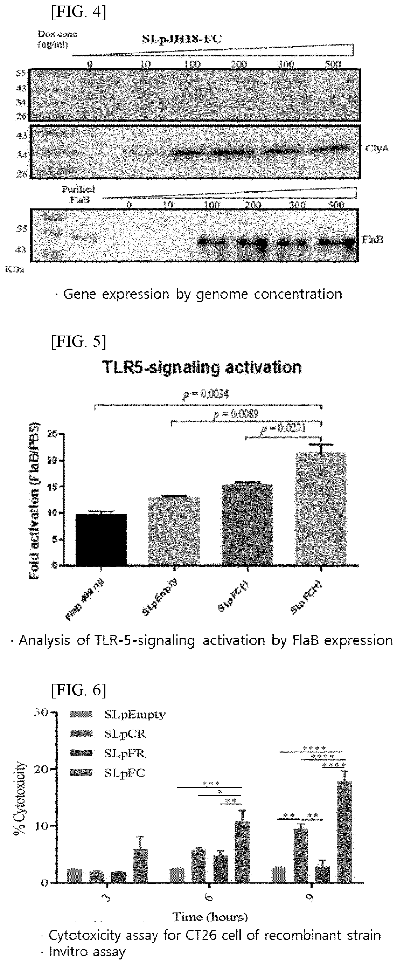

To confirm FlaB expression of the recombinant strain, the activation of TLR-5 signaling caused by FlaB was confirmed. First, TLR-5 signal activation was measured for FlaB 40 ng and SLpEmpty strain as a control, and the SLpFC recombinant strain prepared in Preparation Example 3 was divided into cases without doxycycline (-) and cases with administration (+) and cultured. Then, TLR-5 signal activation was measured and shown in FIG. 4.

As shown in FIG. 4, the SLpFC recombinant strain has activated TLR-5 signal compared to the control group, and in particular, the recombinant strain (+) cultured under the administration of doxycycline showed a significantly higher degree of TLR-5 signal activation. Thus it was confirmed that the FlaB expression level of the recombinant strain was remarkable when doxycycline was administered.

### [1-4] Determination of FlaB and ClyA Expression Levels According to Doxycycline Concentration

In order to confirm the expression levels of FlaB and ClyA in the recombinant strain according to doxycycline concentration, Western blot analysis was performed. 0, 10, 100, 200, 300, and 500 ng/ml of doxycycline were respectively administered to the culture medium of the SLpJH18-FC recombinant strain. Then, ClyA (34 KDa) and FlaB (43KDa) secreted by the recombinant strain up to 2 hours after the mid-log phage subculture were performed by Western blotting, and the results are shown in FIG. 5.

As shown in FIG. 5, as the concentration at which doxycycline was administered increased to 0, 10, 100, 200, 300, and 500 ng/ml, the expression levels of ClyA (34KDa) and FlaB (43 KDa) increased. It was confirmed that the expression levels of ClyA and FlaB of the recombinant strain can be controlled by adjusting the concentration of doxycycline, and it could be seen that the expression and activity ratios could be relatively balanced.

### [Example 2] Confirmation of Anticancer Effect of Recombinant Strain (1)

In order to confirm the anticancer effect of the recombinant strain, cytotoxicity tests of the CT26 cell line were conducted *in vitro.* After treating the SLpCR, SLpFR and SLpFC strains prepared in Preparation Example 3 and the SLpEmpty strain used as a control with CT26, the cytoplasmic enzyme of necrotic cell death (LDH) released from the damaged CT26 was analyzed and shown in FIG. 6.

As shown in FIG. 6, the cytotoxic effect of the SLpFC recombinant strain expressing ClyA and FlaB was remarkable compared to other recombinant strains and the control group, confirming that the anticancer effect of the SLpFC recombinant strain was remarkable.

### [Example 3] Confirmation of Cancer-Targeting Effect of Recombinant Strain

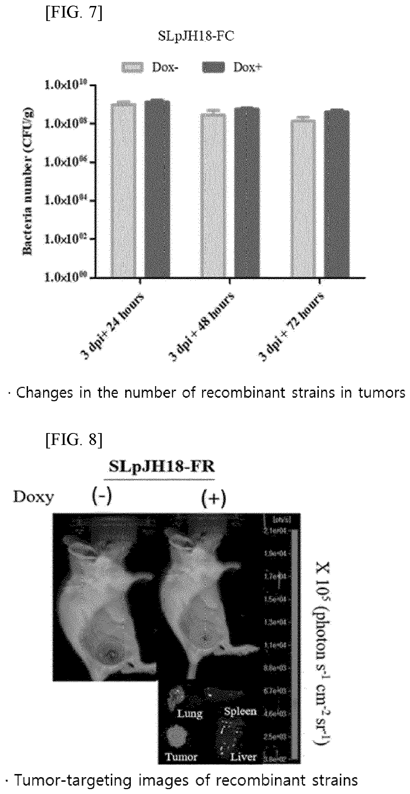

In order to confirm the cancer-targeting effect of the recombinant strain, an *in vivo* experiment was conducted to confirm the number of recombinant strains in the tumor and the targeting image. The attenuated SLpJH18-FC and SLpJH18-FR recombinant strains were intravenously administered to the CT26 mouse model of Preparation Example 4 at 1×10⁷ CFU, and doxycycline was orally administered daily at 3 dpi, while doxycycline was not administered to the control group. As a result, the number of SLpJH18-FC strains in the tumors was measured and shown in FIG. 7, and the fluorescence images expressed by the SLpJH18-FR strains in the tumors were confirmed and shown in FIG. 8.

As shown in FIGS. 7 and 8, the recombinant strain specifically targets cancer cells by confirming that 1×10⁸ CFU or more of the recombinant strain is present in the tumor regardless of doxycycline administration, and that it is present specifically in the tumor.

### [Example 4] Confirmation of Anticancer Effect of Recombinant Strain (2)

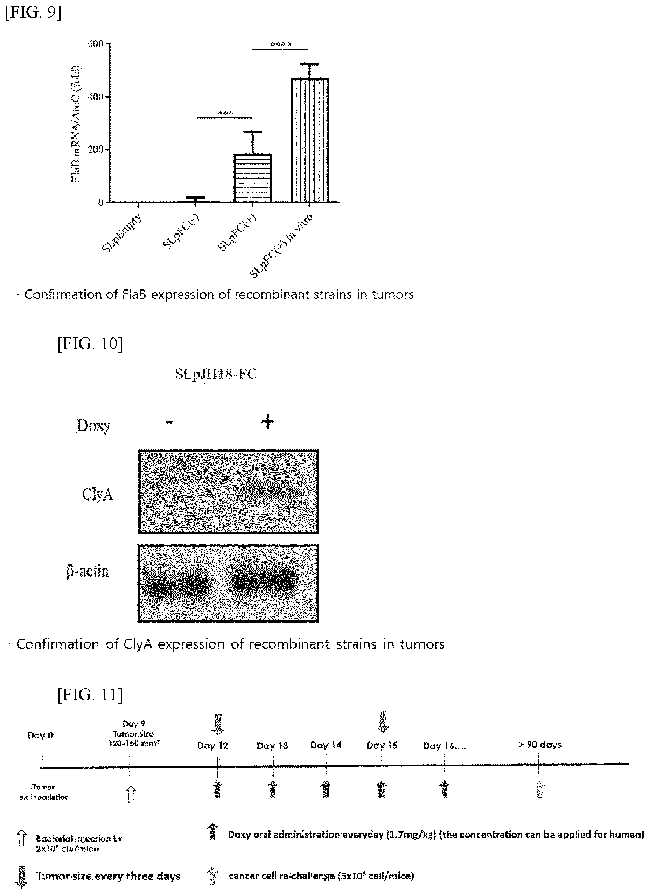

In order to confirm the anticancer effect of the recombinant strain, *in vivo* experiments were conducted to confirm the expression levels of FlaB and ClyA of the SLpJH18-FC recombinant strain in tumors. The attenuated SLpJH18-FC recombinant strain was intravenously administered to the CT26 mouse model of Preparation Example 4 at 1×10⁷ CFU, and doxycycline was orally administered daily at 3 dpi, while doxycycline was not administered to the control group. As a result, the amount of FlaB expression in the tumor is shown in FIG. 9, and the amount of ClyA expression in the tumor is shown in FIG. 10.

As shown in FIGS. 9 and 10, when doxycycline was administered, the expression levels of FlaB and ClyA significantly increased. When the dose of doxycycline was adjusted after administration of the SLpJH18-FC recombinant strain, the expression levels of FlaB and ClyA could be controlled.

### [Example 5] Confirmation of Anticancer Effect of Recombinant Strain (3)

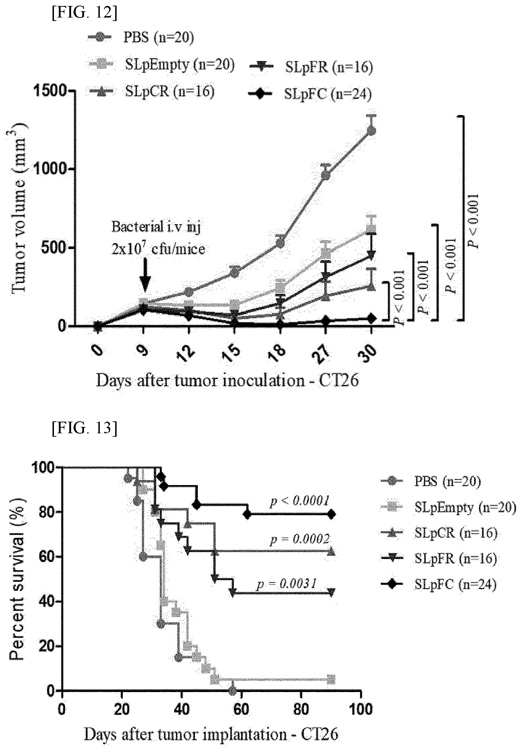

In order to confirm the anticancer effect of the recombinant strain, an *in vivo* experiment was conducted in the same manner as shown in FIG. 11 to confirm the growth inhibitory effect of the SLpJH18-FC recombinant strain on the CT26 cell line in the tumor. The attenuated SLpJH18-FC recombinant strain was intravenously administered to the CT26 mouse model of Preparation Example 4 at 1×10⁷ CFU, and doxycycline was orally administered daily at 1.7mg/kg, while doxycycline was not administered to the control group. The resulting size of the tumor is shown in FIG. 12, and the survival rate of mice is shown in FIG. 13.

As shown in FIGS. 12 and 13, when the SLpJH18-FC recombinant strain was administered, the tumor suppression ability and mouse survival rate were significantly increased compared to the control group, confirming the anticancer effect of the SLpJH18-FC recombinant strain.

### [Example 6] Confirmation of Anticancer Effect of Recombinant Strain (4)

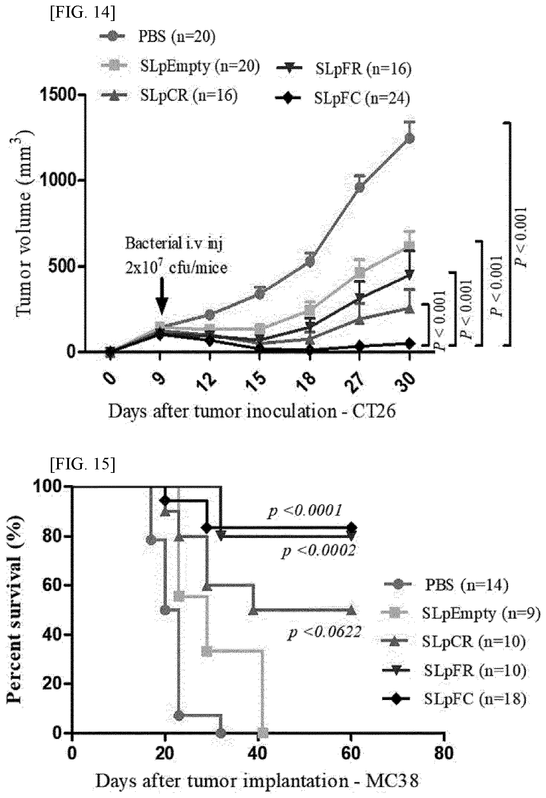

In order to confirm the anticancer effect of the recombinant strain, an *in vivo* experiment was conducted in the same manner as in FIG. 11 to confirm the growth inhibitory effect of the SLpJH18-FC recombinant strain on the MC38 cell line in the tumor. The attenuated SLpJH18-FC recombinant strain was intravenously administered to the CT26 mouse model of Preparation Example 4 at 1×10⁷ CFU, and doxycycline was orally administered daily at 1.7mg/kg, while doxycycline was not administered to the control group. The resulting size of the tumor is shown in FIG. 14, and the survival rate of mice is shown in FIG. 15.

As shown in FIGS. 14 and 15, when the SLpJH18-FC recombinant strain was administered, the tumor suppression ability and mouse survival rate significantly increased compared to the control group, confirming the anticancer effect of the SLpJH18-FC recombinant strain.

### [Example 7] Confirmation of Cancer Recurrence Inhibitory Effect of Recombinant Strain

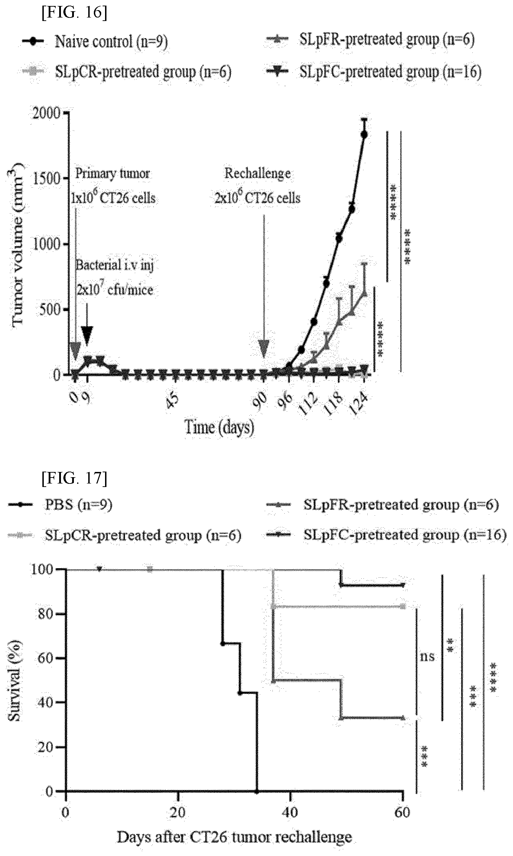

In order to confirm the cancer recurrence inhibitory effect of the recombinant strain, an *in vivo* experiment was conducted in the same manner as shown in FIG. 11 to confirm the growth inhibitory effect of the SLpJH18-FC recombinant strain on the CT26 cell line in the tumor. The attenuated SLpJH18-FC recombinant strain was intravenously administered to the CT26 mouse model of Preparation Example 4 at 1×10⁷ CFU, and doxycycline was orally administered daily at 1.7mg/kg to completely cure the cancer. After 90 days, CT26 cells were re-administered. The size of the re-administered tumors is shown in FIG. 16, and the survival rate of mice is shown in FIG. 17.

As shown in FIGS. 16 and 17, when the SLpJH18-FC recombinant strain was administered, the tumor growth inhibitory effect of re-administration was remarkable compared to the control group, and the survival rate of mice increased significantly, confirming the cancer recurrence inhibition effect of the SLpJH18-FC recombinant strain.

### [Example 8] Confirmation of Cancer Metastasis Inhibitory Effect of Recombinant Strain

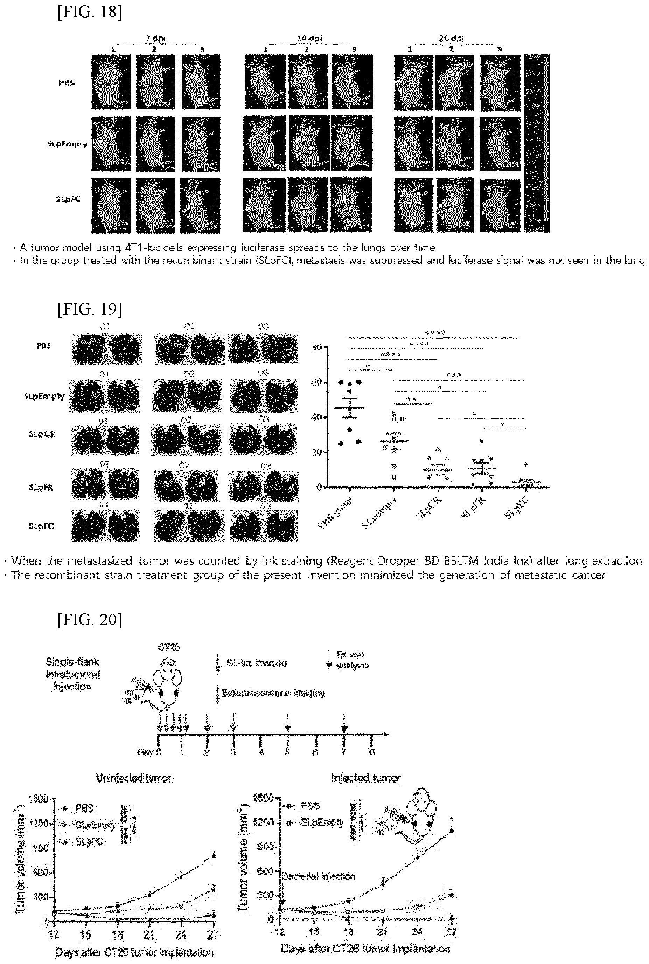

In order to confirm the cancer metastasis inhibitory effect of the recombinant strain, *in vivo* experiments were conducted to confirm the growth inhibitory effect of the SLpJH18-FC recombinant strain on the 4T1-Luc cell line in tumors. A 4T1-Luc cell line expressing luciferase was prepared, and a mouse model injected with the 4T1-Luc cell line was prepared. Thereafter, the attenuated SLpJH18-FC recombinant strain was intravenously administered at 1×10⁷ CFU, and doxycycline was orally administered at 1.7mg/kg daily. The resulting location of the tumor was photographed and shown in FIG. 18, and after removing the lungs the number of tumors metastasized to the lungs were measured and shown in FIG. 19.

As shown in FIGS. 18 and 19, when the SLpJH18-FC recombinant strain was administered, the tumor did not metastasize to the lung compared to the control group, and thus the tumor metastasis suppression effect of the SLpJH18-FC recombinant strain as remarkable.

### [Example 9] Confirmation of Anticancer Effect of Recombinant Strain (5)

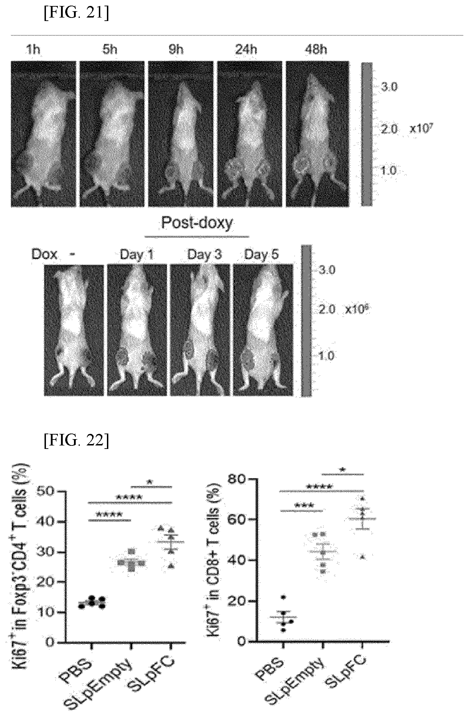

In order to confirm the anticancer effect of the recombinant strain, an *in vivo* experiment was conducted to confirm the Abscopal Effect of the SLpJH18-FC recombinant strain on the CT26 cell line in the tumor. A mouse model in which CT26 was injected into both thighs was prepared and the attenuated SLpJH18-FC recombinant strain was treated on only one side, and doxycycline was administered orally daily. The resulting sizes of both tumors were measured and shown in FIG. 20, tumor images were taken and shown in FIG. 21, and the Ki67 level of mouse T cells was measured and shown in FIG. 22.

As shown in FIGS. 20 and 21, even when the SLpJH18-FC recombinant strain was administered to only one tumor, it was found to have an inhibitory effect on the remaining tumors. In addition, as shown in FIG. 22, when the SLpJH18-FC recombinant strain was treated, Ki67 level increased and immune cells were activated, confirming that the SLpJH18-FC recombinant strain had a remarkable anticancer effect.

Through the above results, in the case of the plasmid comprising the promoter of the present disclosure, it is possible to control the expression of the regulatory protein present downstream of the promoter, ultimately controlling the expression level of anticancer genes present downstream of the tetA and tetR promoters.

Although the present disclosure has been described in detail with reference to the specific features, it will be apparent to those skilled in the art that this description is only of a preferred embodiment thereof, and does not limit the scope of the present disclosure. Thus, the substantial scope of the present disclosure will be defined by the appended claims and equivalents thereto.

Through the above results, in the case of the plasmid comprising the promoter of the present disclosure, it is possible to control the expression of the regulatory protein present downstream of the promoter, ultimately controlling the expression level of anticancer genes present downstream of the tetA and tetR promoters.

Although the present disclosure has been described in detail with reference to the specific features, it will be apparent to those skilled in the art that this description is only of a preferred embodiment thereof, and does not limit the scope of the present disclosure. Thus, the substantial scope of the present disclosure will be defined by the appended claims and equivalents thereto.

## Claims

1. A DNA construct comprising a gene encoding a first protein and a second protein; and a first promoter and a second promoter corresponding thereto,
wherein the first protein is flagellin and the second protein is a toxin protein.

2. The DNA construct of claim 1, wherein the flagellin is flagellin A or flagellin B.

3. The DNA construct of claim 1, wherein the toxin protein is at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), streptolysin O (SLO), pneumolysin (PLO) listeriolysin (LLO), and cytolysin A (ClyA).

4. The DNA construct of claim 1, further comprising a gene encoding a regulatory protein.

5. The DNA construct of claim 1, wherein the first promoter is a tetA promoter, and the second promotor is a tetR promoter.

6. The DNA construct of claim 4, wherein the regulatory protein is a TetR protein.

7. A recombinant vector comprising the DNA construct of any one of claims 1 to 6.

8. A strain into which the recombinant vector of claim 7 has been introduced.

9. The strain of claim 8, wherein the strain is at least one selected from the group consisting of a *Salmonella* sp. strains, *Clostridium* sp. strains, *Bifidobacterium* sp. strains, and *E. coli* strains.

10. A pharmaceutical composition for preventing or treating cancer comprising the strain of claim 8 as an active ingredient.

11. The pharmaceutical composition of claim 10, wherein the cancer is at least one selected from the group consisting of melanoma, fallopian tube cancer, brain cancer, small intestine cancer, esophageal cancer, lymph gland cancer, gallbladder cancer, blood cancer, thyroid cancer, endocrine cancer, oral cancer, liver cancer, biliary tract cancer, colon cancer, rectal cancer, cervical cancer, ovarian cancer, kidney cancer, stomach cancer, duodenum Cancer, prostate cancer, breast cancer, brain tumor, lung cancer, undifferentiated thyroid cancer, uterine cancer, colon cancer, bladder cancer, ureter cancer, pancreatic cancer, bone/soft tissue sarcoma, skin cancer, non-Hodgkin's lymphoma, Hodgkin's lymphoma, multiple myeloma, leukemia, myelodysplastic syndrome , acute lymphoblastic leukemia, acute myeloid leukemia, chronic lymphocytic leukemia, chronic myelogenous leukemia and solitary myeloma.

12. The pharmaceutical composition of claim 10, wherein the pharmaceutical composition inhibits growth or metastasis of cancer.

13. A strain into which the recombinant vector individually comprising one DNA constructs including a gene encoding a first protein and a first promoter corresponding thereto, and other DNA constructs including a gene encoding a second protein and a second promoter corresponding thereto,
wherein the first protein is flagellin and the second protein is a toxin protein.

14. A pharmaceutical composition for preventing or treating cancer comprising the strain of claim 13 as an active ingredient.

15. A pharmaceutical composition for preventing or treating cancer comprising as active ingredients,
flagellin or a nucleotide encoding the same; and
a toxin protein or a nucleotide encoding the same.

16. A strain of claim 8 or claim 13 for preventing or treating cancer.

17. A method for preventing or treating cancer by administering an effective amount of the strain of claim 8 or claim 13 to a target subject.

18. A method for preventing or treating cancer by administering effective amounts of a strain expressing exogenous flagellin and a strain expressing exogenous toxin protein to a subject in combination.
